# EUROPÄISCHE PATENTSCHRIFT

(11) **EP 0 582 288 B1**
(45) Veröffentlichungstag und Bekanntmachung des Hinweises auf die Patenterteilung: **21.10.1998**
(21) Anmeldenummer: 93112495.2
(22) Anmeldetag: 04.08.1993
(51) Int. Cl.: C07D 239/52

(54) **Verfahren zur Herstellung von 2-Halogen-4,6-dialkoxypyrimidinen**
Process for the preparation of 2-halogeno-4,6-dialkoxy pyrimidines
Procédé de préparation de 2-halogéno-4,6-dialkoxypyrimidines

(30) Priorität: 05.08.1992 CH 2458/92
(43) Veröffentlichungstag der Anmeldung: 09.02.1994
(73) Patentinhaber: LONZA AG, CH-3945 Gampel/Wallis (CH)
(72) Erfinder: Escher, André, Dr., Glis (Kanton Wallis) (CH); Previdoli, Felix, Dr., Brig (Kanton Wallis) (CH); Imwinkelried, René, Dr., Brig-Glis (Kanton Wallis) (CH)
(74) Vertreter: Weinhold, Peter, Dr.

(56) Entgegenhaltungen:
- EP-A- 0 024 200
- EP-A- 0 100 425
- EP-A- 0 271 834
- EP-A- 0 424 849
- EP-A- 0 476 554
- DE-A- 3 939 965
- FR-A- 2 562 070
- US-A- 3 225 077
- CHEMICAL ABSTRACTS, vol. 61, no. 5, 31. August 1964, Columbus, Ohio, US; abstract no. 6301b,

## Beschreibung

Die Erfindung betrifft ein neues Verfahren zur Herstellung von 2-Halogen-4,6-dialkoxypyrimidinen der allgemeinen Formel 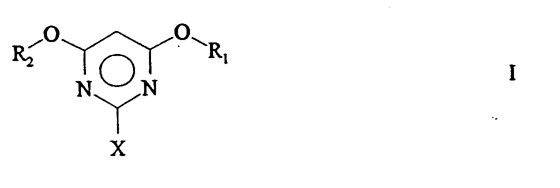 worin X ein Halogenatom, R₁ und R₂ gleich oder verschieden sind und eine C₁-C₄-Alkylgruppe bedeuten, ausgehend von einem Propandiimidat oder dessen Salzen.

2-Halogen-4,6-dialkoxypyrimidine sind beispielsweise wichtige Zwischenprodukte zur Herstellung von 2-Phenoxypyrimidin-Derivaten mit herbizider Wirkung (JP-A 03 200 784).

Eine bekannte Ausführungsform zur Herstellung eines 2-Halogen-4,6-dialkoxypyrimidin-Derivats ist von J.A. Bee & F.L. Rose, J. Chem. Soc., 1966, S. 2031 - 2038 beschrieben. Bei diesem Verfahren wird das 2-Halogen-4,6-dialkoxypyrimidin-Derivat 2-Chlor-4,6-dimethoxypyrimidin durch Diazotierung von 2-Amino-4,6-dimethoxypyrimidin mit Natriumnitrit und anschliessender Hydrolyse mit konzentrierter Salzsäure synthetisiert. Ein grosser Nachteil dieses Verfahrens besteht darin, dass das 2-Chlor-4,6-dimethoxypyrimidin in sehr schlechter Ausbeute erhalten wird.

Aufgabe der Erfindung war, diesen Nachteil zu beseitigen und ein einfaches und wirtschaftliches Verfahren zur Herstellung von 2-Halogen-4,6-dialkoxypyrimidin-Derivaten zur Verfügung zu stellen, wobei das Produkt in guter Ausbeute erhalten wird.

Diese Aufgabe wurde mit dem neuen Verfahren gemäss Patentanspruch 1 gelöst.

Erfindungsgemäss wird das Verfahren derart durchgeführt, dass man in der ersten Stufe ein Propandiimidat oder dessen Salze der allgemeinen Formel 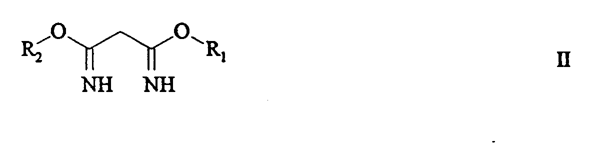 worin R₁ und R₂ die genannte Bedeutung haben mit Cyanamid der Formel

H₂N―CN III

in Gegenwart einer Base bei pH-Werten oberhalb von pH 7 in ein Cyanimidat der allgemeinen Formel 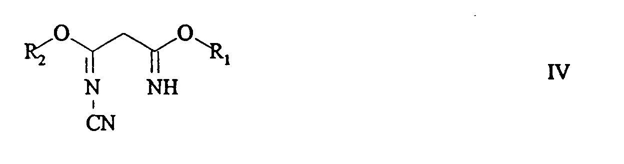 worin R₁ und R₂ die genannte Bedeutung haben, überführt und dieses dann in der zweiten Stufe mit einem Halogenwasserstoff in das Endprodukt gemäss Formel I überführt.

Die Edukte des erfindungsgemässen Verfahrens die Propandiimidate (Formel II) oder deren Salze können nach bekannten Methoden ausgehend von Malonsäuredinitril hergestellt werden (Mc Elvain & Schroeder, J. Am. Chem. Soc., 1949, 71, S. 40).

Die zweckmässigen Reste R₁ und R₂, der Propandiimidate, die gleich oder verschieden sein können, sind eine Methyl-, Ethyl-, Propyl- oder Butylgruppe. Vorzugsweise wird als Propandiimidat 1,3-Dimethylpropandiimidat (R₁ = R₂ = eine Methylgruppe) angewendet.

Zweckmässig wird die erste Stufe des erfindungsgemässen Verfahrens derart durchgeführt, dass man das Propandiimidat oder dessen Salze zu einer Lösung von Base und Cyanamid (Formel III) zugibt und zwar derart, dass sich der pH-Wert auf oberhalb von pH 7 einstellt.

Als Salze der Propandiimidate können Monohydrohalogenid- oder Dihydrohalogenidsalze wie beispielsweise die Salze der Fluor-, Chlor- oder Bromwasserstoffsäure angewendet werden. Vorzugsweise wird das Dihydrochloridsalz angewendet.

Als Basen können beispielsweise Alkali- oder Erdalkalihydrogencarbonate, - carbonate, -hydroxide oder -alkoholate verwendet werden.

Vorzugsweise wird als Base ein Alkalihydrogencarbonat wie beispielsweise Natrium-oder Kaliumhydrogencarbonat insbesondere Kaliumhydrogencarbonat angewendet.

Zweckmässig wird die Base entweder aequimolar oder in leichtem Überschuss bezogen auf das Propandiimidat eingesetzt, vorzugsweise in einer Menge von 1 bis 1,1 mol pro mol Propandiimidat.

Das Cyanamid kann aequimolar oder auch in leichtem Überschuss bezogen auf das Propandiimidat eingesetzt werden. Vorzugsweise wird das Cyanamid in einer Menge von 1 bis 1,5 mol pro mol Propandiimidat angewendet.

Zweckmässig wird die Umsetzung in der ersten Stufe bei einem pH-Wert von 7 bis 12, vorzugsweise von 7 bis 9, durchgeführt.

Die Umsetzung in der ersten Stufe wird zweckmässig bei einer Temperatur von - 25 bis 150°C, vorzugsweise von 0 bis 50°C durchgeführt.

Geeignete Lösungsmittel für die erste Stufe sind beispielsweise Wasser, Ketone wie Aceton oder Methylisobutylketon; Alkohole wie Methanol oder Ethanol; Kohlenwasserstoffe wie Toluol oder Xylol; halogenierte Kohlenwasserstoffe wie Dichlormethan oder 1,2-Dichlorethan; Ester wie Methylacetat oder Ethylacetat. Es können auch Mischungen dieser Lösungsmittel angewendet werden.

Vorzugsweise wird als Lösungsmittel Wasser angewendet.

Nach einer üblichen Umsetzungsdauer von 1 bis 10 h, kann dann das Cyanimidat der allgemeinen Formel IV fachmännisch üblich isoliert werden.

In der zweiten Stufe wird das Cyanimidat mit einem Halogenwasserstoff in das Endprodukt gemäss Formel I überführt.

Als Halogenwasserstoff können Chlorwasserstoff, Bromwasserstoff oder Jodwasserstoff angewendet werden. Vorzugsweise wird Chlorwasserstoff oder Bromwasserstoffverwendet.

Der Halogenwasserstoff kann in einer Menge von 2 bis 20 mol pro mol Cyanimidat angewendet werden. Vorzugsweise wird der Halogenwasserstoff dem Reaktionsgefäss gasförmig bis zur Sättigung eingeleitet.

Die Temperaturen in der zweiten Stufe liegen zweckmässig zwischen -50 und 50°C, vorzugsweise zwischen -20 und 10°C.

Geeignete Lösungsmittel für die zweite Stufe sind inerte organische Lösungsmittel wie beispielsweise Tetrahydrofuran, Toluol, Acetonitril, Methylenchlorid oder niedrig siedende Alkohole. Vorzugsweise wird Tetrahydrofuran oder Toluol verwendet.

Nach einer üblichen Umsetzungsdauer von 1 bis 10 h, kann das 2-Halogen-4,6-dialkoxypyrimidin der allgemeinen Formel I nach fachmännisch üblichen Methoden isoliert werden.

### Beispiel 1

### Herstellung von 2-Chlor-4,6-dimethoxypyrimidin

10 g Kaliumhydrogencarbonat wurden in 50 ml Wasser gelöst und auf 0°C abgekühlt. Danach wurden 5,58 g Cyanamid dazugegeben. Der pH-Wert lag bei 8,3. Anschliessend wurde bei 0°C während ca. 7 Minuten 20,3 g Dimethylpropandiimidat-dihydrochlorid unter CO₂-Entwicklung zugegeben. Nach Ende der Zugabe wurde auf Raumtemperatur erwärmt, wobei aus der gelblich, klaren Lösung ein Feststoff ausfiel. Nach 4 h bei Raumtemperatur wurde der ausgefallene Feststoff abgenutscht und mit 20 ml Wasser gewaschen. Trocknung des Filterrückstandes im Vakuum bei 20°C während 2 Tagen ergab 11,17 g 3-Amino-3-methoxy-N-cyano-2-propenimidat als weisser Feststoff, welcher unter N₂-Strom in 50 ml Tetrahydrofuran suspendiert und auf -20°C abgekühlt wurde. Die Suspension wurde bei -20°C bis -15°C mit total 40 g HCl-Gas gesättigt. Nach 5 h HCl-Einleitung wurde das Reaktionsgemisch auf 5 bis 10°C erwärmt und am Rotavapor total eingeengt. Der Eindampfrückstand wurde in 50 ml Wasser aufgenommen, gerührt und abgenutscht. Der Filterrückstand wurde 3 mal mit 50 ml Wasser chloridfrei gewaschen und im Vakuum bei 35°C über Nacht getrocknet.
Es wurden 10,12 g 2-Chor-4,6-dimethoxypyrimidin als weisse Kristalle erhalten (GC 98,4%), entsprechend einer Ausbeute von 57% bez. auf eingesetztes Dimethylpropandiimidat-dihydrochlorid.

### Beispiel 2

### Herstellung von 2-Brom-4,6-dimethoxypyrimidin

Die Herstellung von 3-Amino-3-methoxy-N-cyano-2-propenimidat (1. Stufe) wurde analog zu Beispiel 1 durchgeführt. 5 g 3-Amino-3-methoxy-N-cyano-2-propenimidat wurden in 50 ml Toluol vorgelegt. Die Suspension wurde auf -5°C gekühlt. Anschliessend wurde bei dieser Temperatur während 3 Stunden HBr-Gas in die Reaktionssuspension eingeleitet. Zur Aufarbeitung wurde die Mischung am Rotationsverdampfer eingeengt, der Rückstand in Ethylacetat aufgenommen und mit Wasser gewaschen. Die organische Phase wurde über Na₂SO₄ getrocknet und eingedampft. Man erhielt 0,67 g Produkt

## Patentansprüche

1. Verfahren zur Herstellung von 2-Halogen-4,6-dialkoxypyrimidinen der allgemeinen Formel 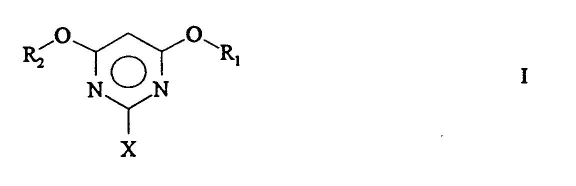 worin X ein Halogenatom, R₁ und R₂ gleich oder verschieden sind und eine C₁-C₄-Alkylgruppe bedeuten, dadurch gekennzeichnet, dass man in der ersten Stufe ein Propandiimidat oder dessen Salze der allgemeinen Formel 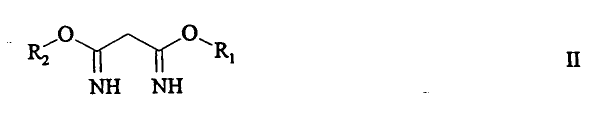 worin R₁ und R₂ die genannte Bedeutung haben mit Cyanamid der Formel
H₂N―CN III
in Gegenwart einer Base bei pH-Werten oberhalb von pH 7 in ein Cyanimidat der allgemeinen Formel 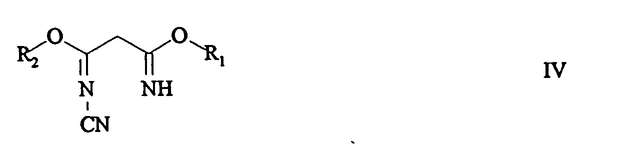 worin R₁ und R₂ die genannte Bedeutung haben, überführt und dieses dann in der zweiten Stufe mit einem Halogenwasserstoff in das Endprodukt gemäss Formel I überführt.

2. Verfahren nach Patentanspruch 1, dadurch gekennzeichnet, dass man als Propandiimidat in der ersten Stufe 1,3-Dimethylpropandiimidat oder dessen Salze anwendet.

3. Verfahren nach mindestens einem der Patentansprüche 1 und 2, dadurch gekennzeichnet, dass man als Base in der ersten Stufe ein Alkalihydrogencarbonat anwendet.

4. Verfahren nach mindestens einem der Patentansprüche 1 bis 3, dadurch gekennzeichnet, dass man die Umsetzung in der ersten Stufe bei einem pH-Wert von 7 bis 12 durchführt.

5. Verfahren nach mindestens einem der Patentansprüche 1 bis 4, dadurch gekennzeichnet, dass man die Umsetzung in der ersten Stufe bei einer Temperatur von - 25 bis 150°C durchführt.

6. Verfahren nach mindestens einem der Patentansprüche 1 bis 5, dadurch gekennzeichnet, dass man als Halogenwasserstoff in der zweiten Stufe Chlorwasserstoff oder Bromwasserstoff anwendet.

7. Verfahren nach mindestens einem der Patentansprüche 1 bis 6, dadurch gekennzeichnet, dass man die Umsetzung in der zweiten Stufe bei einer Temperatur von - 50 bis 50°C durchführt.

## Claims

1. A method for preparing 2-halogen-4,6-dialkoxypyrimidines of the general formula 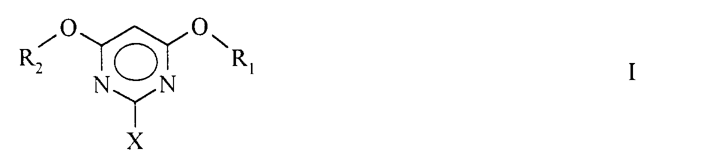 wherein X designates a halogen atom, R₁ and R₂ are identical or different and designate a C₁-C₄ alkyl group, characterised in that, in the first stage, a propanediimidate or the salts thereof having the general formula 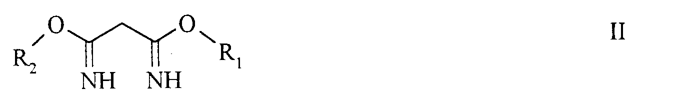 are transformed with cyanamide having the formula
H₂N―CN III
in the presence of a base at pH values exceeding pH 7 into a cyanoimidate having the general formula 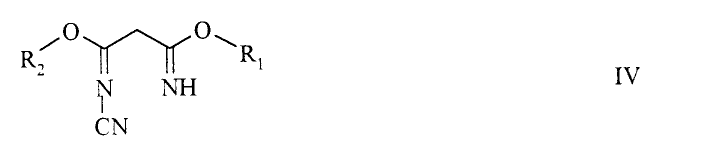 wherein R₁ and R₂ have the named meaning, with the latter then, in the second stage, being transformed by means of a hydrogen halide into the final product in accordance with Formula I.

2. The method according to claim 1, characterised in that the propanediimidate utilised in the first stage is 1,3-dimethylpropanediimidate or the salts thereof.

3. The method according to at least one of claims 1 and 2, characterised in that the base utilised in the first stage is an alkali hydrogen carbonate.

4. The method according to at least one of claims 1 to 3, characterised in that reaction in the first stage is performed at a pH value of 7 to 12.

5. The method according to at least one of claims 1 to 4, characterised in that reaction in the first stage is performed at a temperature from -25 to 150°C.

6. The method according to at least one of claims 1 to 5, characterised in that the hydrogen halide utilised in the second stage is hydrogen chloride or hydrogen bromide.

7. The method according to at least one of claims 1 to 6, characterised in that reaction in the second stage is performed at a temperature from -50 to 50°C.

## Revendications

1. Procédé pour la préparation de 2-halogéno-4,6-dialcoxypyrimidines de la formule générale 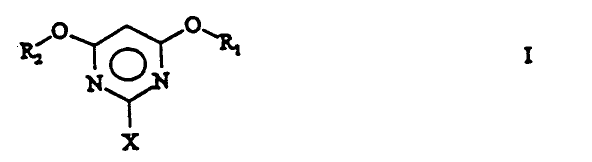 dans laquelle X signifie un atome halogène, R₁ et R₂ sont identiques ou différents et signifient un groupe alkyle en C₁-C₄, caractérisé en ce que dans une première étape, on transforme un propandiimate ou ses sels de la formule générale 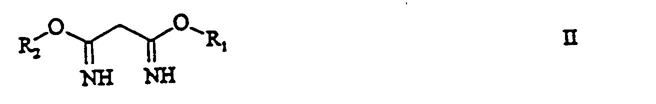 dans laquelle R₁ et R₂ ont la signification citée avec le cyanamide de la formule
H₂N―CN III
en présence d'une base à des valeurs de pH au-dessus de pH 7 dans un cyanimidate de la formule générale 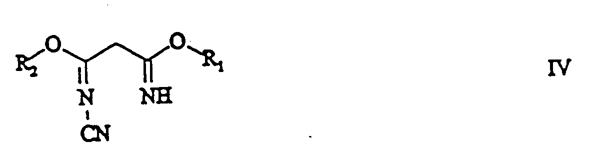 dans laquelle R₁ et R₂ ont la signification citée, et on transforme celui-ci dans la deuxième étape avec un hydrogène halogéné en le produit final selon la formule I.

2. Procédé selon la revendication 1, caractérisé en ce qu'en tant que propandiimidate, on utilise dans la première étape du 1,3-diméthylpropandiimidate ou ses sels.

3. Procédé selon au moins l'une des revendications 1 et 2, caractérisé en ce qu'en tant que base, on utilise dans la première étape, un hydrogénocarbonate alcalin.

4. Procédé selon au moins l'une des revendications 1 à 3, caractérisé en ce que l'on procède à la réaction dans la première étape avec un pH de 7 à 12.

5. Procédé selon au moins l'une des revendications 1 à 4, caractérisé en ce que l'on procède à la réaction dans la première étape à une température de -25 jusqu'à 150°C.

6. Procédé selon au moins l'une des revendications 1 à 5, caractérisé en ce que l'on utilise en tant qu'hydrogène halogéné dans la deuxième étape, un chlorure d'hydrogène ou un bromure d'hydrogène.

7. Procédé selon au moins l'une des revendications 1 à 6, caractérisé en ce que dans la deuxième étape, on conduit la réaction à une température de -50 jusqu'à 50°C.
